(19) Europäisches Patentamt
European Patent Office
Office européen des brevets

(11) EP 4 434 525 A1

(12) # EUROPEAN PATENT APPLICATION
published in accordance with Art. 153(4) EPC

(43) Date of publication:
**25.09.2024 Bulletin 2024/39**

(21) Application number: **22892780.2**

(22) Date of filing: **08.11.2022**

(51) International Patent Classification (IPC):
***A61K 31/4965*** *(2006.01)* ***A61K 9/70*** *(2006.01)*
***A61K 47/32*** *(2006.01)* ***A61P 9/12*** *(2006.01)*

(52) Cooperative Patent Classification (CPC):
**A61K 9/70; A61K 31/4965; A61K 47/32; A61P 9/12**

(86) International application number:
**PCT/JP2022/041600**

(87) International publication number:
**WO 2023/085282 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.11.2021 JP 2021185910**

(71) Applicants:
- **artience Co., Ltd.**
  **Chuo-ku**
  **Tokyo 104-0031 (JP)**
- **Toyochem Co., Ltd.**
  **Chuo-ku**
  **Tokyo 1048379 (JP)**
- **TOA Eiyo Ltd.**
  **Tokyo 104-0032 (JP)**

(72) Inventors:
- **KATO Yutaka**
  **Tokyo 104-8379 (JP)**
- **KOMODA Toshikazu**
  **Tokyo 104-8379 (JP)**
- **NISHIDA Naohiro**
  **Fukushima-shi, Fukushima 960-0280 (JP)**

(74) Representative: **Ter Meer Steinmeister & Partner Patentanwälte mbB**
**Nymphenburger Straße 4**
**80335 München (DE)**

(54) **ADHESIVE PATCH CONTAINING SELEXIPAG AS ACTIVE INGREDIENT**

(57) Provided is an adhesive patch having excellent percutaneous absorption of selexipag. The adhesive patch of the present invention is characterized by including a backing and an adhesive layer that is integrally laminated on one surface of the backing and includes selexipag and an acrylic adhesive. In the adhesive layer, selexipag and an acrylic adhesive are used in combination to provide an adhesive patch having excellent percutaneous absorption of selexipag. The adhesive patch can percutaneously administer selexipag as a drug.

EP 4 434 525 A1

## Description

### Technical Field

The present invention relates to an adhesive patch for percutaneous administration of selexipag as a drug.

### Background Art

Pulmonary hypertension (PH) is a progressive disease with a poor prognosis. This disease causes dysfunction of the heart and lungs due to increased blood pressure in the pulmonary arteries that carry blood from the heart to the lungs. Selexipag (2-{4-[(5,6-diphenylpyrazin-2-yl) (propan-2-yl)amino]butoxy}-N-methanesulfonylacetamide) is known as a therapeutic drug for pulmonary hypertension. Selexipag has been clinically used as an injectable or oral drug.

However, an injectable drug requires hospital visits each time it is administered, placing a burden on the patient. Furthermore, an oral drug requires frequent administration and has restrictions on administration timing, such as needing to be administered after a meal, and these requirements and restrictions place a burden on the patient. Furthermore, an oral preparation may cause side effects due to a rapid increase in the blood concentration of the drug. Thus, there is a desire to solve the problems with conventional injectable drugs and oral preparations and to reduce the burden on patients.

Solutions include a method of administering a drug to the human body through the skin by applying an adhesive patch to the skin. The adhesive patch can be applied to the skin by a patient him/herself. As a result, the drug becomes easy to administer. Furthermore, the number of administrations is reduced, restrictions on administration timing are mitigated, and side effects are reduced as there is only a mild increase in the blood concentration.

Furthermore, besides selexipag, riociguat and the like are known as therapeutic drugs for pulmonary hypertension. Patent Literature 1 discloses an adhesive patch including riociguat.

### Citation List

#### Patent Literature

PTL1: Japanese Patent No. 6459148

### Summary of Invention

#### Technical Problem

However, selexipag has low percutaneous absorption, and as a result, an adhesive patch including selexipag has not been put into practical use.

Thus, an object of the present invention is to provide an adhesive patch having excellent percutaneous absorption of selexipag.

#### Solution To Problem

An adhesive patch of the present invention is characterized by including a backing and an adhesive layer that is integrally laminated on one surface of the backing and includes selexipag and an acrylic adhesive.

[Adhesive layer]

An adhesive patch of the present invention includes a backing and an adhesive layer integrally laminated on one surface of the backing. The adhesive layer includes selexipag and an acrylic adhesive.

(Selexipag)

Selexipag comes in a free base form and an acid addition salt form. The adhesive layer may include at least one of selexipag in the free base form and selexipag in the acid addition salt form. Of these, selexipag in the free base form (free form) is preferable. Selexipag in the free base form refers to selexipag (2-{4-[(5,6-diphenylpyrazin-2-yl)(propan-2-yl)amino]butoxy}-N-methanesulfonylacetamide) itself, which is not in a salt form. With selexipag in the free base form, it is possible to provide an adhesive patch having more excellent percutaneous absorption of selexipag. The adhesive layer may include only one of selexipag in the free base form and selexipag in the acid addition salt form, or both selexipag

in the free base form and selexipag in the acid addition salt form.

Selexipag in the acid addition salt form is a physiologically acceptable acid addition salt of selexipag in the free base form. The physiologically acceptable acid addition salt is not particularly limited. However, examples thereof include: an inorganic acid salt such as a hydrochloride, a hydrobromide, a nitrate, a sulfate, and a phosphate; and an organic acid salt such as a formate, an acetate, a trifluoroacetate, an ascorbate, a benzoate, a cinnamate, a citrate, a fumarate, a glutamate, a tartrate, an oxalate, a glutarate, a camphorate, an adipate, a sorbate, a lactate, a maleate, a linoleate, a linolenate, a malate, a malonate, a mandelate, a methanesulfonate (mesylate), a phthalate, a salicylate, a stearate, an isostearate, a succinate, a propionate, a butyrate, a pamoate, a p-toluenesulfonate (tosylate), and a benzenesulfonate (besylate). Selexipag in the acid addition salt form may be used singly or in combination of two or more types thereof.

The content ratio of selexipag in the adhesive layer is preferably 0.5% by mass or more, more preferably 1% by mass or more, more preferably 2% by mass or more, more preferably 3% by mass or more, more preferably 4% by mass or more, and more preferably 5% by mass or more, relative to 100% by mass of the total amount of selexipag and the acrylic adhesive. Furthermore, the content ratio of selexipag in the adhesive layer is more preferably 30% by mass or less, more preferably 20% by mass or less, more preferably 15% by mass or less, more preferably 10% by mass or less, more preferably 8% by mass or less, and more preferably 6% by mass or less, relative to 100% by mass of the total amount of selexipag and the acrylic adhesive. Setting the content ratio of selexipag in the adhesive layer to 0.5% by mass or more can rapidly increase the blood concentration of selexipag to a desired range. Setting the content ratio of selexipag in the adhesive layer to 30% by mass or less can reduce excessive precipitation of excessive selexipag as crystals in the adhesive layer and can maintain the percutaneous absorption and storage stability of selexipag at high levels.

Note that, in the adhesive patch of the present invention, when calculating the content ratio, the mass percent, and mole percent (e.g., molar ratio, etc.) of each constituent component included in the adhesive layer, the mass of selexipag obtained by converting selexipag in the acid addition salt form to selexipag in the free base form is used as the mass of selexipag in the acid addition salt form. The mass of selexipag obtained by converting selexipag in the acid addition salt form to selexipag in the free base form is defined as the mass of selexipag in the free base form in a molar amount equal to that of selexipag in the acid addition salt form.

(Acrylic adhesive)

The adhesive layer includes an acrylic adhesive. In the adhesive layer, combined use of selexipag and an acrylic adhesive can provide an adhesive patch achieving enhanced percutaneous absorption of selexipag.

The acrylic adhesive preferably contains an acrylic polymer. The acrylic polymer is not particularly limited, and those used as an acrylic adhesive in conventional adhesive patches can be used. The acrylic polymer may be used singly or in combination of two or more types thereof.

The acrylic polymer is preferably a polymer containing a (meth)acrylate monomer unit. The (meth)acrylate monomer is not particularly limited, and examples thereof include an alkyl (meth)acrylate, a hydroxyalkyl (meth)acrylate, and a glycidyl (meth)acrylate. Among these, an alkyl (meth)acrylate is preferable. It is preferable that the (meth)acrylate monomer does not have an amide structure represented by the formula (1) described later. (Meth)acrylate means acrylate or methacrylate.

That is, the acrylic polymer preferably contains an alkyl (meth)acrylate unit. The alkyl (meth)acrylate unit has a moderate affinity for selexipag and can dissolve some or all of the blended selexipag, which may lead to facilitation of the transfer of the selexipag into the skin. Therefore, the acrylic polymer containing the alkyl (meth)acrylate unit can provide an adhesive patch achieving enhanced percutaneous absorption of selexipag.

The alkyl group in the alkyl (meth)acrylate is preferably a linear or branched alkyl group. The number of carbon atoms in the alkyl group of the alkyl (meth)acrylate is preferably 1 to 16, more preferably 1 to 14, more preferably 2 to 12, and particularly preferably 2 to 10.

Examples of the alkyl (meth)acrylate include methyl (meta)acrylate, ethyl (meta)acrylate, n-propyl (meth)acrylate, isopropyl (meta)acrylate, n-butyl (meth)acrylate, isobutyl (meth)acrylate, hexyl (meth)acrylate, n-octyl (meth)acrylate, isoctyl (meta)acrylate, 2-ethylhexyl (meth)acrylate, decyl (meth)acrylate, dodecyl (meth)acrylate, tridecyl (meth)acrylate, hexadecyl (meth)acrylate, cyclododecyl (meth)acrylate, and cyclohexyl(meth)acrylate. Among these, ethyl (meth)acrylate, n-butyl (meth)acrylate, n-octyl (meth)acrylate, and 2-ethylhexyl (meth)acrylate are preferable, ethyl (meth)acrylate, n-octyl (meth)acrylate, and 2-ethylhexyl (meth)acrylate are more preferable, and ethyl acrylate, n-octyl acrylate, and 2-ethylhexyl acrylate are more preferable. The alkyl (meth)acrylate may be used singly or in combination of two or more types thereof.

The acrylic polymer may be a homopolymer of a (meth)acrylate monomer. In such a case, the acrylic polymer is preferably a homopolymer of the alkyl (meth)acrylate. The homopolymer of the alkyl (meth)acrylate can provide an adhesive patch having excellent percutaneous absorbability of selexipag.

In the homopolymer of the alkyl (meth)acrylate, the alkyl (meth)acrylate may contain two or more types of alkyl

(meth)acrylates in combination. Examples of the preferred combination of two or more types of alkyl (meth)acrylates include a combination of 2-ethylhexyl (meth)acrylate and ethyl (meth)acrylate, a combination of ethyl (meth)acrylate and n-octyl (meth)acrylate, and a combination of 2-ethylhexyl (meth)acrylate, ethyl (meth)acrylate and n-butyl (meth)acrylate.

The acrylic polymer may be a copolymer of a (meth)acrylate monomer and another vinyl-based monomer.

Here, the vinyl-based monomer means a monomer having an ethylenically unsaturated double bond.

The acrylic polymer preferably contains a vinyl-based monomer (I) unit having an amide structure represented by the following formula (1).

[Chemical formula 1]

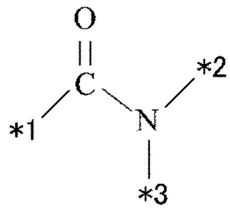

(1)

(In the formula (1), *1, *2, and *3 are each a bond.)

The amide structure represented by the above-mentioned formula (1) is a monovalent, divalent, or trivalent atomic group. The valence of an atomic group means the number (valence) of other atoms or atomic groups to which the atomic group can be bonded. For example, when the amide structure represented by the above-mentioned formula (1) is a monovalent atomic group, the above-mentioned amide structure can be bonded to one other atom or atomic group.

The bonds *1 to *3 in the amide structure represented by the above-mentioned formula (1) are each a single bond, and at least one bond among the bonds *1 to *3 is bonded to a carbon atom that forms an ethylenically unsaturated double bond (carbon-carbon double bond) of the vinyl-based monomer (I) directly or via a plurality of atoms.

In the amide structure represented by the above-mentioned formula (1), when one or two bonds among the bonds *1 to *3 are bonded to a carbon atom that forms an ethylenically unsaturated double bond (carbon-carbon double bond) of the vinyl-based monomer (I) directly or via a plurality of atoms, the remaining bonds may be bonded to a monovalent atom such as a hydrogen atom or a monovalent atomic group such as an alkyl group.

In the amide structure represented by the above-mentioned formula (1), two bonds among the bonds *1 to *3 may be bonded to each other via an atom or an atomic group to form a ring structure. When a ring structure is formed, it is preferable that the bond *1 and the bond *2 or *3 are bonded to each other to form a ring structure.

The storage temperature may change during storage of the adhesive patch. Even if the storage temperature of the adhesive patch changes, it is preferable that the dissolution state and the precipitation state of selexipag in the adhesive layer remain unchanged before and after storage. The reason for this preference is that when the dissolution state and the precipitation state of selexipag change, the percutaneous absorption of selexipag also changes, and there is a possibility that the expected medicinal effect will not be achieved and that such a change will affect the medical treatment. In particular, excessive precipitation of selexipag crystals due to changes in the storage temperature of the adhesive patch can result in non-uniform or reduced percutaneous absorption of the drug.

The vinyl-based monomer (I) having an amide structure represented by the above-mentioned formula (1) has a high affinity for selexipag and has an effect of stabilizing the dissolved state of selexipag. In the present invention, using the acrylic polymer containing the above-mentioned vinyl-based monomer (I) unit can stably maintain the state of selexipag in the adhesive layer. Therefore, even if the storage temperature of the adhesive patch changes, it is possible to reduce the change in the dissolution state and the precipitation state of selexipag in the adhesive layer. As a result, it also becomes possible to reduce the change in percutaneous absorption of selexipag. Using the acrylic polymer containing the above-mentioned vinyl-based monomer (I) unit can provide an adhesive patch having not only excellent percutaneous absorption of selexipag but also excellent storage stability of selexipag.

The acrylic polymer preferably contains the vinyl-based monomer (I) unit having an amide structure represented by the above-mentioned formula (1). That is, the acrylic polymer is preferably a polymer composed of monomers that include the vinyl-based monomer (I) having an amide structure represented by the above-mentioned formula (1).

Examples of the vinyl-based monomer (I) include vinyl-based monomers represented by the following formula (2).

[Chemical formula 2]

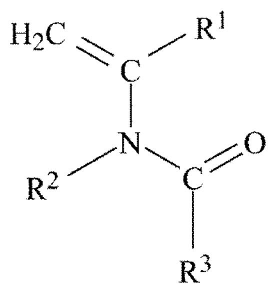

(2)

(In the formula (2), $R^1$ represents a hydrogen atom or a methyl group, $R^2$ and $R^3$, which may be the same as or different from each other, are each an alkyl group , and $R^2$ and $R^3$ may be bonded to each other to form a ring structure.)

In the above-mentioned formulae (2), $R^2$ and $R^3$ may be the same as or different from each other and are each an alkyl group. The alkyl group may be linear or branched. The number of carbon atoms in the alkyl group is preferably 1 to 5. Examples of the alkyl group include a methyl group, an ethyl group, an n-propyl group, an isopropyl group, an n-butyl group, an isobutyl group, a sec-butyl group, and a tert-butyl group. Among these, a methyl group, an ethyl group, and an n-propyl group are preferable, and a methyl group and an ethyl group are more preferable.

In the above-mentioned formula (2), $R^2$ and $R^3$ are preferably bonded to each other to form a ring structure together with a nitrogen atom and a carbon atom to which respective $R^2$ and $R^3$ are bonded.

Specific examples of the vinyl-based monomer represented by the above-mentioned formula (2) include N-vinyl-2-pyrrolidone, N-vinylpiperidone, and N-vinyl-ε-caprolactam. The vinyl-based monomer represented by the above-mentioned formula (2) may be used singly or in combination of two or more types thereof.

Examples of the vinyl-based monomer (I) include vinyl-based monomers represented by the following formula (3).

[Chemical formula 3]

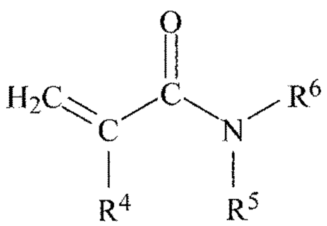

(3)

(In the above-mentioned formula (3), $R^4$ represents a hydrogen atom or a methyl group, $R^5$ represents a hydrogen atom or a methyl group, and $R^6$ represents an alkyl group, an alkoxyalkyl group, or a group represented by the following formula (4),

[Chemical formula 4]

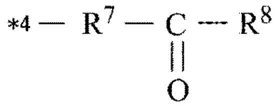

(4)

in the formula (4), *4 represents a bond (single bond), $R^7$ represents an alkylene group, and $R^8$ represents an alkyl group.)

The alkyl group represented by $R^6$ of the above-mentioned formula (3) may be linear or branched. The number of carbon atoms in the alkyl group is preferably 1 to 15, and more preferably 1 to 10. Examples of the alkyl group include a methyl group, an ethyl group, an n-propyl group, an isopropyl group, an n-butyl group, an isobutyl group, a sec-butyl group, a tert-butyl group, a pentyl group, an isopentyl group, a neopentyl group, a hexyl group, a heptyl group, an octyl group, a nonyl group, a decyl group, an undecyl group, a dodecyl group, and an isooctyl group.

The alkoxyalkyl group represented by $R^6$ of the above-mentioned formula (3) may be linear or branched. The number of carbon atoms in the alkoxyalkyl group is preferably 2 to 5. Examples of the alkoxyalkyl group include a methoxymethyl group, a methoxyethyl group, a methoxypropyl group, an ethoxymethyl group, an ethoxyethyl group, an ethoxypropyl group, a propoxymethyl group, a propoxyethyl group, an isopropoxymethyl group, an isopropoxyethyl group, a butoxymethyl group, a sec-butoxymethyl group, an isobutoxymethyl group, and a tert-butoxymethyl group.

When $R^6$ in the above-mentioned formula (3) is the group represented by the above-mentioned formula (4), it is preferable that in the above-mentioned formula (4), the bond *4 is a single bond, $R^7$ is a linear or branched alkylene group, and $R^8$ is a linear or branched alkyl group.

When $R^6$ in the above-mentioned formula (3) is the group represented by the above-mentioned formula (4), the bond *4 in the group represented by the above-mentioned formula (4) is directly bonded to a nitrogen atom forming the amide structure of the vinyl-based monomer represented by the formula (3).

$R^7$ is preferably a linear or branched alkylene group. The number of carbon atoms in the alkylene group is preferably 1 to 10, and more preferably 1 to 6. Examples of the alkylene group include a methylene group, an ethylene group, a methylmethylene group, an ethylmethylene group, a dimethylmethylene group, an ethylmethylmethylene group, an isopropylmethylene group, a trimethylene group, a propylene group, a 1-methyltrimethylene group, a 2-methyltrimethylene group, a tetramethylene group, a dimethylethylene group, and an ethylethylene group. Among these, a dimethylethylene group is preferable.

$R^8$ is preferably a linear or branched alkyl group. The number of carbon atoms in the alkyl group is preferably 1 to 5. Examples of the alkyl group include a methyl group, an ethyl group, an n-propyl group, an isopropyl group, an n-butyl group, an isobutyl group, a sec-butyl group, and a tert-butyl group. Among these, a methyl group, an ethyl group, and an n-propyl group are preferable, and a methyl group and an ethyl group are more preferable.

Specific examples of the vinyl-based monomer represented by the above-mentioned formula (3) include diacetone acrylamide, N-(isobutoxymethyl)acrylamide, N,N-dimethylacrylamide, N,N-dimethylmethacrylamide, N-octylacrylamide, and N-isopropylmethacrylamide. The vinyl-based monomer represented by the above-mentioned formula (3) may be used singly or in combination of two or more types thereof.

As the vinyl-based monomer (I), as described above, the vinyl-based monomer represented by the above-mentioned formula (2) and the vinyl-based monomer represented by the above-mentioned formula (3) are preferable. Among these, N-vinyl-2-pyrrolidone and diacetone acrylamide are more preferable, and N-vinyl-2-pyrrolidone is more preferable. The vinyl-based monomer (I) may be used singly or in combination of two or more types thereof.

The content of the vinyl-based monomer (I) unit in the acrylic polymer is preferably 1% by mass or more, and more preferably 5% by mass or more. The content of the vinyl-based monomer (I) unit in the acrylic polymer is preferably 40% by mass or less, more preferably 35% by mass or less, and more preferably 30% by mass or less. Setting the content of the vinyl-based monomer (I) unit to 1% by mass or more makes it possible to provide an adhesive patch excellent not only in percutaneous absorption of selexipag but also in storage stability of selexipag. On the other hand, setting the content of the vinyl-based monomer (I) unit to 40% by mass or less makes it possible to maintain excellent percutaneous absorption of selexipag.

The acrylic polymer preferably contains the vinyl-based monomer (I) unit having an amide structure represented by the above-mentioned formula (1) and the alkyl (meth)acrylate unit. That is, the acrylic polymer is preferably a copolymer of monomers containing the vinyl-based monomer (I) having an amide structure represented by the above-mentioned formula (1) and the alkyl (meth)acrylate. Such an acrylic polymer makes it possible to provide an adhesive patch having excellent percutaneous absorption and storage stability of selexipag.

When the acrylic polymer contains the vinyl-based monomer (I) unit having an amide structure represented by the above-mentioned formula (1) and the alkyl (meth)acrylate unit, the content of the alkyl (meth)acrylate unit in the acrylic polymer is preferably 60% by mass or more, more preferably 65% by mass or more, and more preferably 70% by mass or more. When the acrylic polymer contains the vinyl-based monomer (I) unit having an amide structure represented by the above-mentioned formula (1) and the alkyl (meth)acrylate unit, the content of the alkyl (meth)acrylate unit in the acrylic polymer is preferably 99% by mass or less, and more preferably 95% by mass or less. Setting the content of the alkyl (meth)acrylate unit to 60% by mass or more makes it possible to enhance the percutaneous absorption of selexipag. Setting the content of the alkyl (meth)acrylate unit to 99% by mass or less makes it possible for the acrylic polymer to contain the vinyl-based monomer (I) unit in a sufficient amount.

The acrylic polymer preferably contains an N-vinyl-2-pyrrolidone unit as the vinyl-based monomer (I) unit and a 2-ethylhexyl (meth)acrylate unit as the alkyl (meth)acrylate unit. The acrylic polymer preferably contains an N-vinyl-2-pyrrolidone unit as the above-mentioned vinyl-based monomer (I) unit, and an ethyl (meth)acrylate unit and an n-octyl (meth)acrylate unit as the alkyl (meth)acrylate units.

Furthermore, the acrylic polymer preferably contains a diacetone acrylamide unit as the above-mentioned vinyl-based monomer (I) unit, and a 2-ethylhexyl (meth)acrylate unit and an n-butyl (meth)acrylate unit as the alkyl (meth)acrylate units.

When the acrylic polymer contains the vinyl-based monomer (I) unit having an amide structure represented by

the above-mentioned formula (1), the mass ratio of the content of selexipag to the content of the vinyl-based monomer (I) unit in the adhesive layer, i.e., [the content (parts by mass) of selexipag / the content (parts by mass) of the vinyl-based monomer (I) unit], is preferably 0.1 or more, more preferably 0.2 or more, and more preferably 0.3 or more. Setting the above-mentioned mass ratio to 0.1 or more makes it possible to provide an adhesive patch having excellent percutaneous absorption of selexipag.

When the acrylic polymer contains the vinyl-based monomer (I) unit having an amide structure represented by the above-mentioned formula (1), the mass ratio of the content of selexipag to the content of the vinyl-based monomer (I) unit in the adhesive layer, i.e., [the content (parts by mass) of selexipag / the content (parts by mass) of the vinyl-based monomer (I) unit], is preferably 1.3 or less, more preferably 1.1 or less, more preferably 0.8 or less, and more preferably 0.7 or less. Setting the above-mentioned mass ratio to 1.3 or less makes it possible to provide an adhesive patch excellent not only in percutaneous absorption of selexipag but also in storage stability of selexipag. In particular, setting the above-mentioned mass ratio to 1.1 or less makes it possible to keep the amount of selexipag remaining in the adhesive patch at the end of administration low, which may lead to prevention of excessive administration of selexipag when removal of the adhesive patch is forgotten and to provision of a highly safe adhesive patch.

The content (parts by mass) of the vinyl-based monomer (I) unit in the adhesive layer is, for example, a value calculated based on the following formula.

$$\text{Content (parts by mass) of the vinyl-based monomer (I) unit in the adhesive layer} = [W_1 \times C_1 + W_2 \times C_2 + \ldots + W_n \times C_n] / 100$$

(In the formula, n is an integer representing the number of types of acrylic polymers contained in the adhesive layer, $W_n$ is the content (parts by mass) of the n-th acrylic polymer in the adhesive layer, and $C_n$ is the content (% by mass) of the vinyl-based monomer (I) unit in the above-mentioned n-th acrylic polymer.)

As described above, the acrylic polymer is preferably a polymer containing a (meth)acrylate monomer unit, and examples of the (meth)acrylate monomer include a hydroxyalkyl (meth)acrylate, a glycidyl (meth)acrylate, and the like in addition to the alkyl (meth)acrylate.

Thus, the acrylic polymer may contain a (meth)acrylate monomer unit, such as a hydroxyalkyl (meth)acrylate unit and a glycidyl (meth)acrylate unit, other than the alkyl (meth)acrylate unit. The (meth)acrylate monomer unit other than the alkyl (meth)acrylate unit may be used singly or in combination of two or more types thereof.

Examples of the hydroxyalkyl (meth)acrylate include hydroxyethyl (meth)acrylate, hydroxypropyl (meth)acrylate, and hydroxybutyl (meth)acrylate. The hydroxyalkyl (meth)acrylate may be used singly or in combination of two or more types thereof.

Furthermore, the acrylic polymer may contain other vinyl-based monomer units in addition to the (meth)acrylate monomer unit and the vinyl-based monomer (I) unit described above. Examples of the other vinyl-based monomers include (meth)acrylic acid, acrylonitrile, vinyl acetate, vinyl chloride, an α-olefin, and styrene. The other vinyl-based monomers may be used singly or in combination of two or more types thereof. Among these, (meth)acrylic acid is preferable. Note that the term "(meth)acrylic acid" means acrylic acid or methacrylic acid.

The content of the (meth)acrylic acid unit in the acrylic polymer is preferably 10% by mass or less, more preferably 5% by mass or less, and more preferably 3% by mass or less. The content of the (meth)acrylic acid unit in the acrylic polymer is preferably 0.1% by mass or more.

The acrylic polymer may be formed by a conventionally known method. Examples thereof include a method of polymerizing the above-described monomers such as (meth)acrylate monomers or vinyl-based monomers (I) in the presence of a polymerization initiator. Specifically, a predetermined amount of monomers, a polymerization initiator, and a polymerization solvent are supplied to a reaction vessel and heated at a temperature of 60 to 80°C for 4 to 48 hours to perform radical polymerization of the monomers.

Examples of the polymerization initiator include an azobis-based polymerization initiator such as 2,2'-azobisisobutyronitrile (AIBN), 1,1'-azobis(cyclohexane-1-carbonitrile), and 2,2'-azobis-(2,4'-dimethylvaleronitrile); and a peroxide-based polymerization initiator such as benzoyl peroxide (BPO), lauroyl peroxide (LPO), and di-tert-butylperoxide. Examples of the polymerization solvent include ethyl acetate, cyclohexane, and toluene. Furthermore, the polymerization reaction is preferably performed under a nitrogen gas atmosphere.

The content of the acrylic polymer in the acrylic adhesive is preferably 80% by mass or more, more preferably 90% by mass or more, more preferably 95% by mass or more, more preferably 98% by mass or more, and particularly preferably 100% by mass. That is, it is particularly preferable that the acrylic adhesive is composed only of an acrylic polymer. Setting the content of the acrylic polymer to 80% by mass or more makes it possible to provide an adhesive

patch having excellent percutaneous absorption of selexipag.

The content ratio of the acrylic adhesive in the adhesive layer is more preferably 70% by mass or more, more preferably 80% by mass or more, more preferably 85% by mass or more, more preferably 90% by mass or more, more preferably 92% by mass or more, and more preferably 94% by mass or more, relative to 100% by mass of the total amount of selexipag and the acrylic adhesive. The content ratio of the acrylic adhesive in the adhesive layer is preferably 99.5% by mass or less, more preferably 99% by mass or less, more preferably 98% by mass or less, more preferably 97% by mass or less, more preferably 96% by mass or less, and more preferably 95% by mass or less, relative to 100% by mass of the total amount of selexipag and the acrylic adhesive. Setting the content ratio of the acrylic adhesive to 70% by mass or more makes it possible to provide an adhesive patch having excellent percutaneous absorption of selexipag. Setting the content ratio of the acrylic adhesive to 99.5% by mass or less makes it possible for the adhesive layer to contain selexipag in a necessary amount.

(Plasticizer)

The adhesive layer preferably contains a plasticizer. Using a plasticizer makes it possible to change the diffusivity of selexipag to adjust the releasability thereof.

Examples of the plasticizer include a fatty acid ester such as methyl laurate, hexyl laurate, isopropyl myristate, myristyl myristate, octyldodecyl myristate, isopropyl palmitate, and cetyl palmitate; an aliphatic alcohol including a monohydric aliphatic alcohol such as myristyl alcohol, cetanol, octyldodecanol, isostearyl alcohol and stearyl alcohol, and a dihydric aliphatic alcohol such as octanediol; a petroleum-based oil such as a liquid paraffin, a paraffinic process oil, a naphthenic process oil, and an aromatic process oil; and a fatty acid amide such as diethanolamide laurate. Among these, a fatty acid ester and a petroleum-based oil are preferable, isopropyl myristate, isopropyl palmitate, and liquid paraffin are more preferable, and isopropyl myristate and isopropyl palmitate are more preferable. The plasticizer may be used singly or in combination of two or more types thereof.

The content of the plasticizer in the adhesive layer is preferably 1 part by mass or more, more preferably 5 parts by mass or more, and more preferably 10 parts by mass or more, relative to 100 parts by mass of the acrylic adhesive. The content of the plasticizer in the adhesive layer is preferably 30 parts by mass or less, more preferably 20 parts by mass or less, and more preferably 15 parts by mass or less, relative to 100 parts by mass of the acrylic adhesive. Setting the content of the plasticizer to 1 part by mass or more makes it possible to change the diffusivity of selexipag and to thereby adjust the releasability thereof. Setting the content of the plasticizer to 30 parts by mass or less makes it possible to keep the adhesiveness of the adhesive layer favorable, and thus to provide an adhesive patch that is easy to handle.

The adhesive layer may contain other additives such as a tackifier and a filler.

(Tackifier)

Examples of the tackifier include a terpene resin, a modified terpene resin, a hydrogenated terpene resin, a terpene phenolic resin, rosin, hydrogenated rosin, a rosin ester, a petroleum resin, a coumarone-indene resin, a phenolic resin, a xylene resin, and an alicyclic saturated hydrocarbon resin. The tackifier may be used singly or in combination of two or more types thereof. The content of the tackifier in the adhesive layer is preferably 15 to 80 parts by mass, and more preferably 20 to 70 parts by mass, relative to 100 parts by mass of the acrylic adhesive.

(Filler)

The filler is used to adjust the shape-restoration properties of the adhesive layer. Examples of the filler include an inorganic filler such as light anhydrous silicic acid, titanium oxide, and zinc oxide; organometallic salts such as calcium carbonate and magnesium stearate; a cellulose derivative such as lactose, crystalline cellulose, ethyl cellulose, and low-substituted hydroxypropylcellulose; and crosslinked polyvinylpyrrolidone. The filler may be used singly or in combination of two or more types thereof. The content of the filler in the adhesive layer is preferably 5 parts by mass or less, and more preferably 0.1 to 2 parts by mass, relative to 100 parts by mass of the acrylic adhesive.

The thickness of the adhesive layer is preferably 20 to 200 μm, more preferably 30 to 150 μm, and particularly preferably 50 to 120 μm. The adhesive layer with the thickness of 20 μm or more can contain selexipag in an amount necessary to obtain a desired medicinal effect. The adhesive layer with the thickness of 200 μm or less can eliminate a requirement where thorough drying must be performed at the time of production in order to reduce the residual solvent in the adhesive layer. Thus, having such a thickness can suppress volatilization or decomposition of selexipag in the adhesive layer.

[Backing]

In the adhesive patch of the present invention, the adhesive layer is integrally laminated on one surface of a backing. The backing is required to have a sufficient material strength for preventing loss of the drug in the adhesive layer and for imparting self-restoration properties to the adhesive patch. Examples of such a backing include resin film, nonwoven fabric, woven fabric, knitted fabric, and aluminum sheet.
Examples of the resin constituting the resin film include a cellulose acetate, rayon, a polyethylene terephthalate, a plasticized vinyl acetate-vinyl chloride copolymer, nylon, an ethylene-vinyl acetate copolymer, a plasticized polyvinyl chloride, a polyurethane, a polyethylene, a polypropylene, and a polyvinylidene chloride. Among these, a polyethylene terephthalate is preferable.
Examples of the material constituting the nonwoven fabric include a polyethylene, a polypropylene, an ethylene-vinyl acetate copolymer, an ethylene-methyl (meth)acrylate copolymer, nylon, a polyester, vinylon, an SIS copolymer, an SEES copolymer, rayon, and cotton, with a polyester being preferred. These materials may be used singly or in combination of two or more types thereof.
The backing may be a single layer or a laminated sheet in which multiple layers are integrally laminated. Examples of the laminated sheet include a laminated sheet in which a polyethylene terephthalate sheet and a nonwoven fabric or a flexible resin film are integrally laminated.
The thickness of the backing is not particularly limited, and is preferably 2 to 200 μm, more preferably 2 to 100 μm.

[Release liner]

In the adhesive patch of the present invention, a release liner may be laminated on one surface of the adhesive layer so as to be freely releasable. The release liner is used to prevent loss of the drug in the adhesive layer and to protect the adhesive layer.
Examples of the release liner include paper and a resin film. Examples of the resin constituting the resin film include a polyethylene terephthalate, a polyethylene, a polypropylene, a polyvinyl chloride, and a polyvinylidene chloride. The surface of the release liner facing the adhesive layer is preferably subjected to a release treatment.

[Production method of adhesive patch]

Examples of a method for producing the adhesive patch of the present invention include the following. (1) In a first method, an adhesive layer-forming solution that includes selexipag, the acrylic adhesive, and a solvent is applied onto one surface of the backing and then dried. As a result, an adhesive layer is integrally laminated on the one surface of the backing. Furthermore, if necessary, a release liner is laminated on the adhesive layer so that a release-treated surface of the release liner faces the adhesive layer. (2) In a second method, the above-mentioned adhesive layer-forming solution is applied onto the release-treated surface of the release liner and then dried to form an adhesive layer on the release liner, and this adhesive layer is then integrally laminated on the backing.
The adhesive layer-forming solution can be obtained by stirring and thereby homogenizing selexipag, the acrylic adhesive and the solvent, and, if necessary, other additives. Examples of the solvent include toluene, normal hexane, cyclohexane, normal heptane, and ethyl acetate. The solvents may be used singly or in combination of two or more types thereof.

Advantageous Effects of Invention

The adhesive patch of the present invention having the above-mentioned configuration is excellent in the percutaneous absorption of selexipag.

Description Of Embodiments

The present invention will be described in more detail below using examples. However, the present invention is not limited to these examples.

[Examples]

Preparation methods or details of the acrylic polymers used as the acrylic adhesives in Examples and Comparative Examples are described below.

(Preparation of acrylic polymer (A1))

A reaction liquid containing monomers including 65 parts by mass of 2-ethylhexyl acrylate and 35 parts by mass of N-vinyl-2-pyrrolidone, and 185 parts by mass of ethyl acetate was supplied to a polymerizer, and the inside of the polymerizer was set to be a nitrogen atmosphere at 80°C. Then, a polymerization initiator solution obtained by dissolving 0.6 parts by mass of lauroyl peroxide in 16 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an acrylic polymer (A1) solution with an acrylic polymer (A1) content of 30% by mass.

(Preparation of acrylic polymer (A2))

A reaction liquid containing monomers including 75 parts by mass of 2-ethylhexyl acrylate and 25 parts by mass of N-vinyl-2-pyrrolidone, and 50 parts by mass of ethyl acetate was supplied to a polymerizer, and the inside of the polymerizer was set to be a nitrogen atmosphere at 80°C. Then, a polymerization initiator solution obtained by dissolving 1.2 parts by mass of lauroyl peroxide in 30 parts by mass of ethyl acetate and 20 parts by mass of cyclohexane was added to the above-mentioned reaction liquid over 24 hours to copolymerize the above-mentioned monomers. After the polymerization was completed, ethyl acetate was further added to the above-mentioned reaction liquid to obtain an acrylic polymer (A2) solution with an acrylic polymer (A2) content of 30% by mass.

(Preparation of acrylic polymer (A3))

A reaction liquid containing monomers including 75 parts by mass of 2-ethylhexyl acrylate, 22 parts by mass of N-vinyl-2-pyrrolidone, and 3 parts by mass of acrylic acid, and 150 parts by mass of ethyl acetate was supplied to a polymerizer, and the inside of the polymerizer was set to be a nitrogen atmosphere at 80°C. Then, a polymerization initiator solution obtained by dissolving 0.6 parts by mass of lauroyl peroxide in 17 parts by mass of ethyl acetate was added to the above-mentioned reaction liquid over 24 hours to copolymerize the above-mentioned monomers. After the polymerization was completed, ethyl acetate was further added to the above-mentioned reaction liquid to obtain an acrylic polymer (A3) solution with an acrylic polymer (A3) content of 30% by mass.

(Preparation of acrylic polymer (A4))

A reaction liquid containing monomers including 10 parts by mass of N-vinyl-2-pyrrolidone, 50 parts by mass of ethyl acrylate, and 40 parts by mass of n-octyl acrylate, and 50 parts by mass of ethyl acetate was supplied to a polymerizer, and the inside of the polymerizer was set to be a nitrogen atmosphere at 80°C. Then, a polymerization initiator solution obtained by dissolving 1 part by mass of lauroyl peroxide in 30 parts by mass of ethyl acetate and 20 parts by mass of cyclohexane was added to the above-mentioned reaction liquid over 24 hours to copolymerize the above-mentioned monomers. After the polymerization was completed, ethyl acetate was further added to the above-mentioned reaction liquid to obtain an acrylic polymer (A4) solution with an acrylic polymer (A4) content of 30% by mass.

(Preparation of acrylic polymer (A5))

A reaction liquid containing monomers including 5 parts by mass of N-vinyl-2-pyrrolidone, 50 parts by mass of ethyl acrylate, and 45 parts by mass of n-octyl acrylate, and 140 parts by mass of ethyl acetate was supplied to a polymerizer, and the inside of the polymerizer was set to be a nitrogen atmosphere at 80°C. Then, a polymerization initiator solution obtained by dissolving 0.4 parts by mass of lauroyl peroxide in 20 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an acrylic polymer (A5) solution with an acrylic polymer (A5) content of 30% by mass.

(Preparation of acrylic polymer (A6))

A reaction liquid containing monomers including 57 parts by mass of 2-ethylhexyl acrylate, 29 parts by mass of n-butyl acrylate, and 14 parts by mass of diacetone acrylamide, and 150 parts by mass of ethyl acetate was supplied to a polymerizer, and the inside of the polymerizer was set to be a nitrogen atmosphere at 80°C. Then, a polymerization initiator solution obtained by dissolving 0.6 parts by mass of lauroyl peroxide in 17 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an

acrylic polymer (A6) solution with an acrylic polymer (A6) content of 30% by mass.

(Preparation of acrylic polymer (A7))

A reaction liquid containing monomers including 75 parts by mass of 2-ethylhexyl acrylate and 25 parts by mass of ethyl acrylate, and 75 parts by mass of ethyl acetate was supplied to a polymerizer, and the inside of the polymerizer was set to be a nitrogen atmosphere at 80°C. Then, a polymerization initiator solution obtained by dissolving 0.6 parts by mass of lauroyl peroxide in 17 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an acrylic polymer (A7) solution with an acrylic polymer (A7) content of 30% by mass.

(Preparation of acrylic polymer (A8))

A reaction liquid containing monomers including 56 parts by mass of ethyl acrylate and 44 parts by mass of n-octyl acrylate, and 122 parts by mass of ethyl acetate was supplied to a polymerizer, and the inside of the polymerizer was set to be a nitrogen atmosphere at 80°C. Then, a polymerization initiator solution obtained by dissolving 0.431 parts by mass of lauroyl peroxide in 20 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an acrylic polymer (A8) solution with an acrylic polymer (A8) content of 30% by mass.

(Preparation of acrylic polymer (A9))

A reaction liquid containing monomers including 57 parts by mass of 2-ethylhexyl acrylate, 14 parts by mass of ethyl acrylate, and 29 parts by mass of n-butyl acrylate, and 75 parts by mass of ethyl acetate was supplied to a polymerizer, and the inside of the polymerizer was set to be a nitrogen atmosphere at 80°C. Then, a polymerization initiator solution obtained by dissolving 0.6 parts by mass of lauroyl peroxide in 17 parts by mass of ethyl acetate was added to the above-mentioned reaction liquid over 14 hours to copolymerize the above-mentioned monomers. After the polymerization was completed, ethyl acetate was further added to the above-mentioned reaction liquid to obtain an acrylic polymer (A9) solution with an acrylic polymer (A9) content of 30% by mass.

· Acrylic polymer (A10)
(acrylic polymer including 68.15% by mass of 2-ethylhexyl acrylate unit, 26.5% by mass of vinyl acetate unit, 5.2% by mass of hydroxyethyl acrylate unit, and 0.15 mass of glycidyl methacrylate unit, manufactured by Henkel AG & Co. KGaA, product name "DURO-TAK 387-2287")

(Examples 1 to 10)

To obtain the adhesive layer that includes each component in the blending amount shown in Table 1, an adhesive layer-forming solution was produced by blending 4.4 parts by mass of selexipag in the free base form and 12.0 parts by mass of isopropyl myristate in a solution including 83.6 parts by mass of each of the acrylic polymers (A1) to (A10).

Next, a 75-μm thick polyethylene terephthalate film that had been subjected to a silicone release treatment was prepared as a release liner. The adhesive layer-forming solution was applied onto the silicone release-treated surface of the polyethylene terephthalate film, and dried at 60°C for 30 minutes to produce a laminate in which the 70-μm. thick adhesive layer was formed on the silicone release-treated surface of the polyethylene terephthalate film. Then, a 38-μm thick polyethylene terephthalate film was prepared as a backing, and one surface of the backing and the adhesive layer of the above-mentioned laminate were stacked together so as to face each other. The adhesive layer of the laminate was then transferred to and integrally laminated on the backing to produce an adhesive patch. The adhesive patch was evaluated for various performance characteristics according to the methods described below. Note that the content of selexipag in the free base form in the resulting adhesive layer was 4.4% by mass.

(Example 11, and Comparative Examples 1 and 2)

To obtain the adhesive layer including each component in the blending amount shown in Table 2, an adhesive layer-forming solution including selexipag in the free base form, the following acrylic polymer (A11), the following rubber adhesives (B1) and (B2), isopropyl myristate, and the following tackifier was produced. Then, an adhesive patch was produced in the same manner as in Example 1 and evaluated for various performance characteristics according to the

methods described below.

· Acrylic polymer (A11) (acrylic polymer including 2-ethylhexyl acrylate unit, acrylic acid unit, n-butyl acrylate unit, and vinyl acetate unit, manufactured by Henkel AG & Co. KGaA, product name "DURO-TAK 387-2051")

- · Rubber adhesive (B1) (styrene-isoprene-styrene block copolymer (SIS), a 1:1.54 mass ratio mixture of product name "KRATON D DX401" and product name "KRATON D D1117PT", manufactured by Kraton Corp.)
- · Rubber adhesive (B2) (polyisobutylene (PIB), a 1:1:1 mass ratio mixture of product name "OPPANOL B100", product name "OPPANOL B30", and product name "OPPANOL B12", manufactured by BASF SE)
- ·Tackifier: alicyclic saturated hydrocarbon resin (manufactured by Arakawa Chemical Industries, Ltd., product name "ARKON P-90")

(Examples 12 and 13)

To obtain the adhesive layer including each component in the blending amount shown in Table 3, the blending amount of the acrylic polymer (A4) was changed, as were the type and blending amount of the plasticizer. Herein, the blending amounts are those relative to 4.4 parts by mass of selexipag in the free base form. Then, an adhesive patch was produced in the same manner as in Example 1 and evaluated for various performance characteristics according to the methods described below.

(Examples 14 to 18)

To obtain the adhesive layer including each component in the blending amount shown in Table 4, the blending amount of isopropyl myristate was set to 12.0 parts by mass, and the blending amount of selexipag in the free base form and the blending amount of the acrylic polymer (A4) were changed. Then, an adhesive patch was produced in the same manner as in Example 1 and evaluated for various performance characteristics according to the methods described below.

(Examples 19 to 22)

To obtain the adhesive layer including each component in the blending amount shown in Table 5, the blending amount of the acrylic polymer (A4) and the blending amount of isopropyl myristate were changed. Herein, the blending amounts are those relative to 4.4 parts by mass of selexipag in the free base form. Then, an adhesive patch was produced in the same manner as in Example 1 and evaluated for various performance characteristics according to the methods described below.

[Evaluation]

For the adhesive patches of Examples and Comparative Examples, skin permeation properties and the dissolution state of selexipag in the free base form were evaluated according to the following procedures.

[Skin permeation test (cumulative skin permeation amount)]

To evaluate skin permeation properties, an adhesive patch that had just been produced and one that had been stored at 40°C for 1 month were subjected to a hairless mouse skin permeation test using the following method. Note that an adhesive patch was stored by sealing the adhesive patch in a light-shielded aluminum bag and keeping the adhesive patch in an atmosphere with a temperature of 40°C for 1 month.

An adhesive patch was cut into a planar circular test piece with a diameter of 1 cm and an area of 0.8 $cm^2$. After a receptor side of a Franz diffusion cell kept at 32°C was filled with a receptor fluid, the skin of a hairless mouse was fixed in the Franz diffusion cell, so that the dermis side of the skin was brought into contact with the receptor fluid. A test piece with the release liner peeled off and removed was attached to the stratum corneum side of this skin using the adhesive layer. Note that the receptor fluid included phosphate buffered saline adjusted to pH 7.4 and polyethylene glycol 400 at a volume ratio (phosphate buffered saline : polyethylene glycol 400) of 6 : 4.

Twenty-four hours after applying the test piece to the skin, the receptor fluid on the underside of the skin was collected, and the concentration of selexipag in the free base form was measured using HPLC. Next, the permeation amount of selexipag in the free base form was calculated from the concentration of selexipag in the free base form and the receptor fluid volume. A value obtained by dividing the thus calculated permeation amount of selexipag in the free base form by the area (0.8 $cm^2$) of the test piece was taken as a 24-h cumulative skin permeation amount ($\mu g/cm^2$) of selexipag in the free base form. The obtained results are shown in the "24-h Cumulative skin permeation amount" column

of Tables 1 to 5.

Furthermore, the theoretical amount of selexipag in the free base form included in the adhesive layer of the test piece was defined as $Q_T$ (μg/cm$^2$), and the 24-h cumulative skin permeation amount of selexipag in the free base form obtained in the skin permeation test described above was defined as $Q_E$ (μg/cm$^2$). Based on the following formula, the cumulative skin permeability (%) of selexipag in the free base form 24 hours after application (hereinafter referred to as "24-h cumulative skin permeability (%)") was calculated.

$$\text{24-h Cumulative skin permeability (\%)} = (Q_E/Q_T) \times 100$$

Note that the theoretical amount $Q_T$ (μg/cm$^2$) of selexipag in the free base form included in the adhesive layer of the test piece was a value calculated as follows. First, using the test piece obtained by cutting an adhesive patch that had just been produced, a mass $W_T$ (μg/cm$^2$) of the adhesive layer per unit area of the above-mentioned test piece was determined by dividing the mass (μg) of the adhesive layer in the above-mentioned test piece by the area (0.8 cm$^2$) of the above-mentioned test piece. Furthermore, the content (% by mass) of selexipag in the free base form in the adhesive layer included in the adhesive patch that had just been produced was defined as $C_s$. Then, a value obtained based on the following formula was defined as the theoretical amount $Q_T$ (μg/cm$^2$) of selexipag in the free base form included in the adhesive layer of the test piece.

$$Q_T\ (\mu g/cm^2) = W_T \times C_s/100$$

Furthermore, the 24-h cumulative skin permeability of selexipag in the free base form in the adhesive patch that had just been produced was defined as $Y_0$, and the 24-h cumulative skin permeability of selexipag in the free base form in the adhesive patch after storage at 40°C for 1 month was defined as $Y_1$. Based on the following formula, a rate in change of the 24-h cumulative skin permeability of selexipag in the free base form was calculated. The results are shown in the "Rate of change" column of Tables 1 to 5.

Rate of change in 24-h cumulative skin permeability of selexipag in free base form = $100 \times (Y_1 - Y_0) / Y_0$

[Dissolution state]

Immediately after production of the adhesive patch, a planar square test piece with an area of 5 cm$^2$ was punched out from the adhesive patch. Next, the release liner was peeled off and removed from the test piece, and the surface of the adhesive layer was observed in a 1 cm$^2$ range approximately in the center of the test piece using a polarizing microscope at 40x magnification to take a photograph. The photograph thus obtained was observed to examine a precipitation state of crystals of selexipag in the free base form. The crystals were each classified into one of "small crystal, medium crystal, and large crystal" shown below depending on the size of the crystal diameter, and the number of crystals of each size is further classified into one of "A, B, and C" shown below for evaluation.

Next, the test piece after observation was sealed in an aluminum package and then stored in a temperature atmosphere of 40°C for 1 month. After that, the test piece was further stored in a temperature atmosphere of 25°C for 24 hours. After storage, the test piece was taken out, and the precipitation state of crystals of selexipag in the free base form was observed immediately in the same manner as in the case of one that had just been produced, and evaluation was performed in the same manner.

<Size of crystal diameter>

Small crystal: crystal with a crystal diameter of 10 μm or more and less than 110 μm.

Medium crystal: crystal with a crystal diameter of 110 μm or more and less than 1000 μm.

Large crystal: crystal with a crystal diameter of 1000 μm or more.

Note that the crystal diameter means the diameter of a perfect circle that has the smallest diameter while being able to surround the crystal in the photograph.

<Number of crystals>

A: the number of crystals was 0.
B: the number of crystals was 1 or more and less than 100.
C: the number of crystals was 100 or more.

The evaluation results are shown in the "Dissolution state" column of Tables 1 to 5. In the above-mentioned column, the number of crystals of each size is described in parentheses in the order of small crystals, medium crystals, and large crystals according to the above-mentioned criteria.

For example,

in a case where there were 0 small crystals, 0 medium crystals, and 0 large crystals, the result was described as (A, A, A),
in a case where there were 0 small crystals, 3 medium crystals, and 0 large crystals, the result was described as (A, B, A), and
in a case where there were 100 or more small crystals, 0 medium crystals, and 0 large crystals, the result was described as (C, A, A).

That is, (A, A, A) means that the entire amount of selexipag in the free base form was dissolved, and no crystals of selexipag in the free base form were precipitated.

Furthermore, after the crystals grow during storage at 40°C for 1 month, the result (A, A, A) of one that had just been produced may change to (A, B, A), the result (A, B, A) of one that had just been produced may change to (A, A, B), and the result (C, A, A) of one that had just been produced may change to (A, C, A).

[Table 1]

| | | | | Example | | | | | | | | | |
|---|---|---|---|---|---|---|---|---|---|---|---|---|---|
| | | | | 1 | 2 | 3 | 4 | 5 | 6 | 7 | 8 | 9 | 10 |
| Adhesive layer blending | Drug | Selexipag in free base form | Blending amount [parts by mass] | 4.4 | | | | | | | | | |
| | | | (% by mass) ※1 | (5.0 % by mass) | | | | | | | | | |
| | Plasticizer | Isopropyl myristate | Blending amount [parts by mass]※ | 12.0 | | | | | | | | | |
| | | | (parts by mass) 2 | (14.4 parts by mass) | | | | | | | | | |
| | Acrylic adhesive | Acrylic polymer | Types※ | (A1) | (A2) | (A3) | (A4) | (A5) | (A6) | (A7) | (A8) | (A9) | (A10) |
| | | | Blending amount [parts by mass] | 83.6 | | | | | | | | | |
| | | | (% by mass) 3 | (95.0 % by mass) | | | | | | | | | |
| Acrylic polymer monomer composition [parts by mass] | N-vinyl-2-pyrrolidone | | | 35 | 25 | 22 | 10 | 5 | 0 | 0 | 0 | 0 | 0 |
| | Diacetone acrylamide | | | 0 | 0 | 0 | 0 | 0 | 14 | 0 | 0 | 0 | 0 |
| | 2-Ethylhexyl acrylate | | | 65 | 75 | 75 | 0 | 0 | 57 | 75 | 0 | 57 | 68.15 |
| | Ethyl acrylate | | | 0 | 0 | 0 | 50 | 50 | 0 | 25 | 56 | 14 | 0 |
| | n-Octyl acrylate | | | 0 | 0 | 0 | 40 | 45 | 0 | 0 | 44 | 0 | 0 |
| | n-Butyl acrylate | | | 0 | 0 | 0 | 0 | 0 | 29 | 0 | 0 | 29 | 0 |
| | Acrylic acid | | | 0 | 0 | 3 | 0 | 0 | 0 | 0 | 0 | 0 | 0 |
| | Vinyl acetate | | | 0 | 0 | 0 | 0 | 0 | 0 | 0 | 0 | 0 | 26.5 |
| | Hydroxyethyl acrylate | | | 0 | 0 | 0 | 0 | 0 | 0 | 0 | 0 | 0 | 5.2 |
| | Glycidyl methacrylate | | | 0 | 0 | 0 | 0 | 0 | 0 | 0 | 0 | 0 | 0.15 |
| Mass ratio [content (parts by mass) of selexlpag in free base form / content (parts by mass) of N-vinyl-2-pyrrolidone unit] | | | | 0.2 | 0.2 | 0.2 | 0.5 | 1.1 | - | - | - | - | - |

(continued)

| | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| Mass ratio [content (parts by mass) of selexipag in free base form / content (parts by mass) of diacetone acrylamide unit] | | | - | - | - | - | - | 0.4 | - | - | - | - |
| Skin permeation test | 24-h Cumulative skin permeation amount ($\mu g/cm^2$) | Immediately after production | 138.0 | 185.2 | 211.0 | 170.3 | 189.8 | 239.8 | 242.3 | 231.7 | 189.5 | 83.8 |
| | | After storage at 40°C for 1 month | 125.2 | 184.7 | 214.1 | 163.3 | 163.0 | 164.0 | 106.7 | 110.2 | 74.2 | 63.6 |
| | 24-h Cumulative skin permeability (%) | Immediately after production | 41.2 | 56.4 | 67.9 | 50.4 | 58.7 | 67.0 | 71.9 | 71.7 | 57.0 | 26.1 |
| | | After storage at 40°C for 1 month | 39.3 | 55.3 | 69.7 | 48.2 | 50.3 | 50.5 | 32.6 | 34.2 | 21.9 | 18.9 |
| | Rate of change | | -5% | -2% | +3% | -4% | -14% | -25% | -55% | -52% | -62% | -28% |
| Dissolution state | | Immediately after production | (A, A, A) | (A, A, A) | (A, A, A) | (A, A, A) | (A, A, A) | (B, A, A) | (A, B, A) | (A, B, A) | (A, B, A) | (C, A, A) |
| | | After storage at 40°C for 1 month | (A, A, A) | (A, A, A) | (A, A, A) | (A, A, A) | (A, B, A) | (A, B, A) | (A, A, B) | (A, A, B) | (A, A, B) | (A, C, A) |

※ 1 Content ratio of selexipag in free base form In 100% by mass of total amount of selexipag in free base form and acrylic adhesive
※ 2 Content of plasticizer relative to 100 parts by mass of acrylic adhesive
※ 3 Content ratio of acrylic adhesive In 100% by mass of total amount of selexipag In free base form and acrylic adhesive

[Table 2]

| | | | | Example | Comparative Example | |
|---|---|---|---|---|---|---|
| | | | | 11 | 1 | 2 |
| Adhesive layer blending | Drug | Selexipag in free base form | Blending amount [parts by mass] | 4.4 | 4.4 | 4A |
| | | | (% by mass) ※ 1 | (5.0 % by mass) | (7.0 % by mass) | (5.0 % by mass) |
| | Plasticizer | Isopropyl myristate | Blending amount [parts by mass]※ | 12.0 | 12.0 | 12.0 |
| | | | (parts by mass) 2 | (14.4 parts by mass) | (20.5 parts by mass) | (14.4 parts by mass) |
| | Tackifier | Alicyclic saturated hydrocarbon resin※ | Blending amount [parts by mass] | 0 | 25.1 | 0 |
| | Acrylic adhesive | Acrylic polymer※ | Type | (A11) | - | - |
| | | | Blending amount [parts by mass] | 83.6 | 0 | 0 |
| | | | (% by mass) 3 | (95.0% by mass) | 0 | 0 |
| | Rubber adhesive | | Type | - | (B1) | (B 2) |
| | | | Blending amount [parts by mass] | 0 | 58.5 | 83.6 |
| | | | (% by mass) 3 | 0 | (93.0 % by mass) | (95.0 % by mass) |
| Mass ratio [content (parts by mass) of selexipag in free base form / content (parts by mass) of N-vinyl-2-pyrrolidone unit] | | | | - | - | - |
| Mass ratio [content (parts by mass) of selexipag in free base form / content (parts by mass) of diacetone acrylamide unit] | | | | - | - | - |
| Skin permeation test | 24-h Cumulative skin permeation amount ($\mu g/cm^2$) | Immediately after production | | 43.0 | 7.6 | 1.0 |
| | | After storage at 40°C for 1 month | | 52.4 | 4.8 | 0.0 |
| | 24-h Cumulative skin permeability (%) | Immediately after production | | 14.2 | 2.3 | 0.3 |
| | | After storage at 40°C for 1 month | | 18.7 | 1.5 | 0.0 |
| | Rate of change | | | +32% | -35% | -100% |

(continued)

| | | Example | Comparative Example | |
|---|---|---|---|---|
| | | 11 | 1 | 2 |
| Dissolution state | Immediately after production | (C, A, A) | (C, A, A) | (C, A, A) |
| | After storage at 40°C for 1 month | (C, A, A) | (C, A, A) | (C, A, A) |

※ 1 Content ratio of selexipag in free base form in 100% by mass of total amount of selexipag in free base form and acrylic adhesive or rubber adhesive
※ 2 Content of plasticizer relative to 100 parts by mass of acrylic adhesive or rubber adhesive
※ 3 Content ratio of acrylic adhesive or rubber adhesive In 100% by mass of total amount of selexipag in free base form and acrylic adhesive or rubber adhesive

[Table 3]

| | | | | Example | |
|---|---|---|---|---|---|
| | | | | 12 | 13 |
| Adhesive layer blending | Drug | Selexipag in free base form※ | Blending amount[parts by mass] | 4.4 | 4.4 |
| | | | (% by mass) 1 | (5.0 % by mass) | (4.5 % by mass) |
| | Plasticizer | Isopropyl myristate※※ | Blending amount[parts by mass] | 0 | 0 |
| | | | (parts by mass) 2 | 0 | 0 |
| | | Isopropyl palmitate※※ | Blending amount[parts by mass] | 12.0 | 0 |
| | | | (parts by mass) 2 | (14.4 parts by mass) | 0 |
| | | Liquid paraffin | Blending amount[parts by mass] | 0 | 3.0 |
| | | | (parts by mass) 2 | 0 | (3.2parts by mass) |
| | Acrylic adhesive | Acrylic polymer (A4) | Blending amount[parts by mass] | 83.6 | 92.6 |
| | | | (% by mass) 3 | (95.0 % by mass) | (95.5 % by mass) |

(continued)

<table>
<tr><td colspan="3" rowspan="2"></td><td colspan="2">Example</td></tr>
<tr><td>12</td><td>13</td></tr>
<tr><td rowspan="3">Acrylic polymer monomer composition [parts by mass]</td><td colspan="2">N-vinyl-2-pyrrolidone</td><td colspan="2">10</td></tr>
<tr><td colspan="2">Ethyl acrylate</td><td colspan="2">50</td></tr>
<tr><td colspan="2">n-Octyl acrylate</td><td colspan="2">40</td></tr>
<tr><td colspan="3">Mass ratio [content (parts by mass) of selexipag in free base form / content (parts by mass) of N-vinyl-2-pyrrolidone units]</td><td colspan="2">0.5</td></tr>
<tr><td rowspan="5">Skin permeation test</td><td rowspan="2">24-h Cumulative skin permeation amount ($\mu$g/cm$^2$)</td><td>Immediately after production</td><td>198.8</td><td>209.0</td></tr>
<tr><td>After storage at 40°C for 1 month</td><td>216.3</td><td>193.1</td></tr>
<tr><td rowspan="2">24-h Cumulative skin permeability (%)</td><td>Immediately after production</td><td>56.8</td><td>59.7</td></tr>
<tr><td>After storage at 40°C for 1 month</td><td>59.4</td><td>55.5</td></tr>
<tr><td colspan="2">Rate of change</td><td>+5%</td><td>-7%</td></tr>
<tr><td colspan="2" rowspan="2">Dissolution state</td><td>Immediately after production</td><td>(A, A, A)</td><td>(A, A, A)</td></tr>
<tr><td>After storage at 40°C for 1 month</td><td>(A, A, A)</td><td>(A, A, A)</td></tr>
<tr><td colspan="5">※ 1 Content ratio of selexlpag in free base form in 100% by mass of total amount of selexipag in free base form and acrylic adhesive<br>※ 2 Content of plasticizer relative to 100 parts by mass of acrylic adhesive<br>※ 3 Content ratio of acrylic adhesive in 100% by mass of total amount of selexipag In free base form and acrylic adhesive</td></tr>
</table>

[Table 4]

| | | | | Example | | | | |
|---|---|---|---|---|---|---|---|---|
| | | | | 14 | 15 | 16 | 17 | 18 |
| Adhesive layer blending | Drug | Selexipag In free base form | Blending amount [parts by mass] | 1.0 | 2.0 | 4.7 | 6.0 | 10.0 |
| | | | (% by mass) ※ 1 | (1.1 % by mass) | (2.3 % by mass) | (5.3 % by mass) | (6.8 % by mass) | (11.4 % by mass) |
| | Plasticizer | Isopropyl myristate | Blending amount [parts by mass]※ | 12.0 | 12.0 | 12.0 | 12.0 | 12.0 |
| | | | (parts by mass) 2※ | (13.8 parts by mass) | (14.0 parts by mass) | (14.4 parts by mass) | (14.6 parts by mass) | (15.4 parts by mass) |
| | Acrylic adhesive | Acrylic polymer (A4) | Blending amount [parts by mass] | 87.0 | 86.0 | 83.3 | 82.0 | 78.0 |
| | | | (% by mass) 3 | (98.9 % by mass) | (97.7 % by mass) | (94.7 % by mass) | (93.2 % by mass) | (88.6 % by mass) |
| Acrylic polymer monomer composition [parts by mass] | N-vlnyl-2-pyrrolldone | | | 10 | | | | |
| | Ethyl acrylate | | | 50 | | | | |
| | n-Octyl acrylate | | | 40 | | | | |
| Mass ratio [content (parts by mass) of selexipag in free base form /content (parts by mass) of N-vinyl-2-pyrrolidone units] | | | | 0.1 | 0.2 | 0.6 | 0.7 | 1.3 |
| Skin permeation test | 24-h Cumulative skin permeation amount ($\mu$g/cm$^2$) | Immediately after production | | 34.4 | 73.9 | 161.6 | 221.5 | 90.2 |
| | | After storage at 40° C for 1 month | | 39.2 | 70.8 | 170.2 | 185.4 | 90.0 |
| | 24-h Cumulative skin permeability (%) | Immediately after production | | 46.9 | 47.3 | 45.9 | 50.8 | 12.0 |
| | | After storage at 40° C for 1 month | | 51.9 | 47.7 | 49.2 | 41.1 | 11.7 |
| | Rate of change | | | +11% | +1% | +7% | -19% | -3% |

(continued)

| | | Example | | | | |
|---|---|---|---|---|---|---|
| | | 14 | 15 | 16 | 17 | 18 |
| Dissolution state | Immediately after production | (A, A, A) | (A, A, A) | (A, A, A) | (A, A, A) | (C, A, A) |
| | After storage at 40° C for 1 month | (A, A, A) | (A, A, A) | (A, A, A) | (A, B, A) | (C, A, A) |
| ※ 1 Content ratio of selexipag in free base form In 100% by mass of total amount of selexipag In free base form and acrylic adhesive<br>※ 2 Content of plasticizer relative to 100 parts by mass of acrylic adhesive<br>※ 3 Content ratio of acrylic adhesive In 100% by mass of total amount of selexipag in free base form and acrylic adhesive | | | | | | |

[Table 5]

| | | | | Example | | | |
|---|---|---|---|---|---|---|---|
| | | | | 19 | 20 | 21 | 22 |
| Adhesive layer blending | Drug | Selexipag in free base form | Blending amount [parts by mass] | 4.4 | 4.4 | 4.4 | 4.4 |
| | | | (% by mass) ※ 1 | (4.4 % by mass) | (4.6 % by mass) | (4.9 % by mass) | (5.2 % by mass) |
| | Plasticizer | Isopropyl myristate | Blending amount [parts by mass]※ | 0.0 | 5.0 | 10.0 | 15.0 |
| | | | (parts by mass) 2※ | (0 parts by mass) | (5.5 parts by mass) | (11.7 parts by mass) | (18.6 parts by mass) |
| | Acrylic adhesive | Acrylic polymer (A4) | Blending amount [parts by mass] | 95.6 | 90.6 | 85.6 | 80.6 |
| | | | (% by mass) 3 | (95.6 % by mass) | (95.4 % by mass) | (95.1 % by mass) | (94.8 % by mass) |
| Acrylic polymer monomer composition [parts by mass] | N-vinyl-pyrrolidone | | | 10 | | | |
| | Ethyl acrylate | | | 50 | | | |
| | n-Octyl acrylate | | | 40 | | | |
| Mass ratio [content (parts by mass) of selexipag in free base form / content (parts by mass) of N-vinyl-2-pyrrolidone units] | | | | 0.5 | 0.5 | 0.5 | 0.5 |

(continued)

| | | | Example | | | |
|---|---|---|---|---|---|---|
| | | | 19 | 20 | 21 | 22 |
| Skin permeation test | 24-h Cumulative skin permeation amount ($\mu g/cm^2$) | Immediately after production | 62.1 | 110.6 | 170.4 | 198.5 |
| | | After storage at 40°C for 1 month | 62.3 | 119.5 | 175.4 | 182.4 |
| | 24-h Cumulative skin permeability (%) | Immediately after production | 18.5 | 314 | 49.1 | 56.6 |
| | | After storage at 40°C for 1 month | 18.7 | 34.5 | 52.9 | 54.3 |
| | Rate of change | | +1% | +10% | +8% | -4% |
| Dissolution state | | Immediately after production | (A, A, A) | (A, A, A) | (A, A, A) | (A, A, A) |
| | | After storage at 40°C for 1 month | (A, A, A) | (A, A, A) | (A, A, A) | (A, A, A) |
| ※ 1 Content ratio of selexipag in free base form in 100% by mass of total amount of selexlpag In free base form and acrylic adhesive<br>※ 2 Content of plasticizer relative to 100 parts by mass of acrylic adhesive<br>※ 3 Content ratio of acrylic adhesive in 100% by mass of total amount of selexipag In free base form and acrylic adhesive | | | | | | |

Industrial applicability

The present invention, as described above, can provide an adhesive patch having excellent percutaneous absorption of selexipag.

(Cross-reference to related application)

The present application claims the priority under Japanese Patent Application No. 2021-185910, filed on November 15, 2021, the disclosure of which is hereby incorporated in its entirety by reference.

## Claims

1. An adhesive patch comprising: a backing; and an adhesive layer that is integrally laminated on one surface of the backing and includes selexipag and an acrylic adhesive.

2. The adhesive patch according to claim 1, wherein the acrylic adhesive contains an acrylic polymer containing a vinyl-based monomer (I) unit having an amide structure represented by the following formula (1);

[Chemical formula 1]

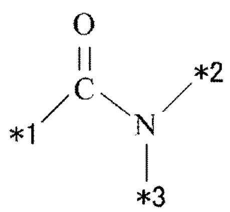

(1)

(in the formula (1), *1, *2, and *3 are each a bond).

**3.** The adhesive patch according to claim 2, wherein the vinyl-based monomer (I) unit includes at least one of an N-vinyl-2-pyrrolidone unit and a diacetone acrylamide unit.

**4.** The adhesive patch according to claim 2 or 3, wherein a mass ratio of a content of selexipag to a content of the vinyl-based monomer (I) unit in the adhesive layer [the content (parts by mass) of selexipag / the content (parts by mass) of the vinyl-based monomer (I) unit] is 0.2 or more and 1.1 or less.

## INTERNATIONAL SEARCH REPORT

International application No.
**PCT/JP2022/041600**

**A. CLASSIFICATION OF SUBJECT MATTER**

***A61K 31/4965***(2006.01)i; ***A61K 9/70***(2006.01)i; ***A61K 47/32***(2006.01)i; ***A61P 9/12***(2006.01)i
FI: A61K31/4965; A61P9/12; A61K9/70 401; A61K47/32

According to International Patent Classification (IPC) or to both national classification and IPC

**B. FIELDS SEARCHED**

Minimum documentation searched (classification system followed by classification symbols)

A61K31/4965; A61K9/70; A61K47/32; A61P9/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | US 2019/0099383 A1 (KYDONIEUS, Agis) 04 April 2019 (2019-04-04)<br>entire text | 1-4 |
| A | CN 112107559 A (PUJI BIOLOGICAL TECHNOLOGY (TAIZHOU) CO., LTD.) 22 December 2020 (2020-12-22)<br>entire text | 1-4 |
| A | JP 2011-51986 A (HISAMITSU PHARMACEUT CO INC) 17 March 2011 (2011-03-17)<br>entire text | 1-4 |
| A | JP 2020-500882 A (CORSAIR PHARMA, INC) 16 January 2020 (2020-01-16)<br>entire text | 1-4 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 December 2022** | **24 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/041600**

| Patent document cited in search report | Publication date (day/month/year) | Patent family member(s) | Publication date (day/month/year) |
|---|---|---|---|
| US 2019/0099383 A1 | 04 April 2019 | (Family: none) | |
| CN 112107559 A | 22 December 2020 | WO 2020/253833 A1 | |
| JP 2011-51986 A | 17 March 2011 | (Family: none) | |
| JP 2020-500882 A | 16 January 2020 | WO 2018/106632 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6459148 B
- JP 2021185910 A